# EUROPÄISCHE PATENTSCHRIFT

(11) **EP 0 804 608 B1**
(45) Veröffentlichungstag und Bekanntmachung des Hinweises auf die Patenterteilung: **02.09.1998**
(21) Anmeldenummer: 95923321.4
(22) Anmeldetag: 13.06.1995
(51) Int. Cl.: C12P 19/00, C07H 1/00, A61K 31/70

(54) **VERFAHREN ZUR HERSTELLUNG VON POLYVALENTEN UND PHYSIOLOGISCH ABBAUBAREN KOHLENHYDRAT-REZEPTORBLOCKERN DURCH ENZYMATISCHE GLYCOSYLIERUNGSREAKTIONEN UND DEREN VERWENDUNG ZUR HERSTELLUNG VON KOHLENHYDRATBAUSTEINEN**
PROCESS FOR PRODUCING POLYVALENT AND PHYSIOLOGICALLY DEGRADABLE CARBOHYDRATE RECEPTOR BLOCKERS BY ENZYMATIC GLYCOSYLATION REACTIONS, AND USES THEREOF FOR MANUFACTURING CARBOHYDRATE COMPONENTS
PROCEDE DE FABRICATION DE BETA-BLOQUANTS DE GLUCIDES POLYVALENTS ET PHYSIOLOGIQUEMENT DEGRADABLES AU MOYEN DE REACTIONS DE GLYCOSYLATION ENZYMATIQUE ET LEUR UTILISATION POUR LA FABRICATION DE CONSTITUANTS DE GLUCIDES

(30) Priorität: 16.06.1994 DE 4420943
(43) Veröffentlichungstag der Anmeldung: 05.11.1997
(73) Patentinhaber: HOECHST AKTIENGESELLSCHAFT, 65929 Frankfurt am Main (DE)
(72) Erfinder: HÖRSCH, Brigitte, D-65830 Kriftel (DE); AHLERS, Michael, D-55127 Mainz (DE); KRETZSCHMAR, Gerhard, D-65760 Eschborn (DE); BARTNIK, Eckart, D-65205 Wiesbaden (DE); SEIFFGE, Dirk, D-55246 Mainz-Kostheim (DE)
(86) Internationale Anmeldenummer: EP9502285
(87) Internationale Veröffentlichungsnummer: WO9534673

(56) Entgegenhaltungen:
- EP-A- 0 123 456
- EP-A- 0 601 417
- J.Cell.Biol. 123/4, 1988, p. 43 - 45

## Beschreibung

Die vorliegende Erfindung betrifft ein Herstellungsverfahren durch enzymatische Glycosylierungsreaktionen für polyvalente Kohlenhydrat-Rezeptorblocker auf Polymerbasis, die in vivo weder in ihrer Gesamtheit noch in Form von Abbauprodukten Unverträglichkeitsreaktionen hervorrufen, sowie ihre Verwendung zur Herstellung von Kohlenhydratbausteinen.

Die Bedeutung der Kohlenhydrate als Informationsträger bei physiologisch relevanten Erkennungsprozessen ist in den letzten Jahren näher untersucht und entschlüsselt worden. Die Anordnung auf der Zelloberfläche in Form von Liganden ermöglicht ihnen, aufgrund ihrer Bindung an spezifische Rezeptoren, eine entscheidende Rolle bei der interzellulären Kommunikation und damit bei interzellulären Erkennungsprozessen zu spielen. Kohlenhydratliganden auf Zelloberflächen sind Erkennungsdomänen für Viren, Bakterien, Toxine und Lectine. Daher spielen sie eine entscheidende Rolle z.B. bei bakteriellen und viralen Infektionen und der Einleitung von Entzündungsprozessen, z.B. rheumatische Arthritis, Allergien, Nachinfarktsyndrom, Schock, Schlaganfall, Sepsis. Untersuchungen haben gezeigt, daß bei entzündlichen Prozessen das von Endothelzellen exprimierte Selektine in vivo die Anhaftung von Leukocyten über einen Kohlenhydratliganden am Entzündungsherd vermittelt.

Von besonderer Bedeutung für die Zelladhäsion sind sialylierte und/oder fucosylierte Kohlenhydrate wie Sialyl-Lewis X und Sialyl-Lewis A.

Die Therapie entzündlicher Erkrankungen mit freien Oligosacchariden, die anstelle der natürlichen Liganden an Rezeptoren binden sollen, scheitert an den sehr hohen Mengen des zu verabreichenden Oligosaccharids, da die Affinität zwischen Rezeptor und Oligosaccharid gering ist (K_{D} ~ 10⁻⁴M bei der Wechselwirkung zwischen einem monovalenten Galactosid und dem entsprechenden Lectin D. T. Connolly et al. J. Biol. Chem. 257, 939, (1982)).

Divalente Strukturen mit z.T. besserer Bindung an den jeweiligen Rezeptor werden von Wong et al. (J. Am. Chem. Soc. 115, 7549 (1993) und in der US 5,254,676 beschrieben.

Es ist außerdem bekannt, daß eine erhöhte Wechselwirkung zwischen Rezeptor und Ligand durch die Kopplung mehrerer Liganden auf einer Oberfläche erreicht wird. Am Beispiel des Virusproteins Hemaglutinin, das an Neuraminsäure auf der Zelloberfläche bindet, konnte gezeigt werden, wie durch Verwendung eines Polymers dieser polyvalente Effekt sich signifikant auf die Wechselwirkung Ligand-Rezeptor auswirkt (monovalent K_{D} = 2x20⁻⁴M, polyvalent K_{D} = 3x10⁻⁷M, A. Spaltenstein et al. J. Am. Chem. Soc. 113, 686 (1991)).

Als Oberflächen wurden bisher Liposomen (N. Yamazaki, Int. J. Biochem. 24, 99 (1991); WO 91/19501; WO 91/19502), Polyacrylamide (R. C. Rathi et al. J. Polym. Sci.: Part A: Polym. Chem. 29, 1895 (1991), S.-I. Nishimura et al. Macromolecules 24· 4236 (1991)), Polylysin oder sulfatierte Polysaccharide verwendet. Diese polyvalenten Strukturen haben den Nachteil entweder in vivo nur wenig stabil oder durch Abbau in toxische Metaboliten in vivo unverträglich zu sein. Bei Polylysin oder sulfatierten Polysacchariden treten unspezifische Wechselwirkungen mit Zelloberflächenstrukturen auf. In den europäischen Offenlegungsschriften 0 089 938, 0 089 939 und 0 089 940 werden Kohlenhydratverbindungen unterschiedlicher Kettenlänge beschrieben, die identisch mit den auf Zelloberflächen befindlichen Liganden bzw den auf Mikroorganismen befindlichen Rezeptoren sind. Der Erfindungsgedanke ist hierbei, die sich auf den Mikroorganismen befindenden Rezeptoren durch die Kohlenhydratverbindungen in vitro und in vivo zu blockieren, um Erkrankungen diagnostizieren und therapieren zu können. Die Kohlenhydratverbindungen können an einen Träger gekoppelt werden. Dieser wird u.a. zur Herstellung von Antikörpern verwendet. In der WO 92/02527 wird ebenfalls ein an einen festen Träger gekoppelter Oligosaccharidbaustein offenbart, welcher dazu dient, entzündliche Prozesse zu diagnostizieren. Der feste Träger verhält sich gegenüber physiologischen Systemen inert, wird also nicht physiologisch abgebaut.

In der EP 0 601 417 A2 wird dagegen ein physiologisch abbaubarer polyvalenter Kohlenhydratrezeptorblocker auf Polymerbasis offenbart, der an der Polymeroberfläche Oligosaccharidbausteine trägt. Eine verbesserte Wirksamkeit als Arzneimittel wird durch die verstärkte Wechselwirkung der sich polyvalent auf der Polymeroberfläche befindenden Kohlenhydratbausteine mit Rezeptoren und durch Blockierung spezifischer Strukturen bewirkt.

Der in der genannten EP 0 601 417 A2 beschriebene Kohlenhydratrezeptorblocker ist physiologisch verträglich und hat vorzugsweise ein Molekulargewicht von <70 kD.

Im einzelnen hat der in der genannten EP 0 601 417 A2 offenbarte, physiologisch verträgliche und pysiologisch abbaubare Kohlenhydratrezeptorblocker auf Polymerbasis folgende Struktur:

Kohlenhydratseitenketten - Spacer - hydrophiles, biodegradables Polymer-(gegebenenfalls) Wirkungsverstärker,
wobei die aus 1 bis 20 natürlich vorkommenden gleichen oder verschiedenen Monosaccharidbausteinen bestehenden Kohlenhydratseitenketten über einen oder mehrere bifunktionelle Spacer natürlicher oder synthetischer Herkunft an ein hydrophiles, biodegradables Polymer gekoppelt sind, wobei das hydrophile, biodegradable Polymer gegebenenfalls noch mit einem Wirkungsverstärker verbunden ist, welcher aus ein oder mehreren Gruppen mit hydrophoben, hydrophilen oder ionischen Eigenschaften besteht, ein Vernetzer oder ein Löslichkeitsverbesserer ist.

Der Kohlenhydratanteil des Kohlenhydratrezeptorblockers gemäß der EP 0 601 417 A2 kann beispielsweise aus den folgenden Zuckerresten bestehen:
Galβ1-4GlcNAc-;
Galβ1-3GlcNAc-;
SA*α*2-6Galβ1-4GlcNAc-;
SA*α*2-3Galβ1-4GlcNAc-;
SA*α*2-3Galβ1-3GlcNAc-;
Galβ1-4(Fucα1-3)GlcNAc-;
Galβ1-3(Fucα1-3)GlcNAc-;
SAα2-3Galβ1-3(Fucα1-4)GlcNAc-;
SAα2-3Galβ1-4(Fucα1-3)GlcNAc-;

Weitere Beispiele für bevorzugte Ausführungsformen des Kohlenhydratanteils sind:
Sialyl-Lewis X, Sialyl-Lewis A, VIM-2 sowie die folgenden Blutgruppendeterminanten Lewis A, B, X, Y und A-Typ¹, A-Typ², B-Typ¹, B-Typ² und H-Typ¹, H-Typ² (R.U. Lemieux, Chem. Soc. Rev., 1978, S. 423 und 1989, S. 347)

Beispiele für die besonders bevorzugte Ausführungsform des Kohlenhydratanteils sind Sialyl-Lewis X, Sialyl-Lewis A oder VIM-2.

Die Formel von Sialyl-Lewis X lautet: NeuNAcα2-3Galβ1-4(Fucα1-3)GlcNAc und von Sialyl-Lewis A: NeuNAcα2-3Galβ1-3(Fucα1-4)GlcNAc. Die Formel von VIM-2 lautet:
NeuNAcα2-3Galβ1-4GlcNAcβ1-3Galβ1-4(Fucα1-3)GlcNAc;

Die genannte EP 0 601 417 A2 offenbart auch ein Verfahren zur Herstellung des Kohlenhydratrezeptorblockers.

Die Synthese des Kohlenhydratrezeptorblockers erfolgt nach dem beschriebenen Verfahren im Labormaßstab. Das bedeutet, der erfindungsgemäße Kohlenhydratrezeptorblocker wird in Milligrammenge bis Grammenge synthetisiert, während die für seine Synthese notwendigen Zwischenprodukte, d.h. das hydrophile biodegradable Polymer, der bifunktionelle Spacer und der Wirkungsverstärker in Gramm- bis Kilogrammenge hergestellt werden können. Eine Ausnahme bildet jedoch der Kohlenhydratanteil, Er kann lediglich in Milligrammenge bis zu einem Gramm synthetisiert werden. Hier sind die literaturbekannten Synthesekonzepte zur Herstellung von Oligosacchariden so angelegt, daß nach einer Reaktion, die üblicherweise nicht mit quantitativer Ausbeute verläuft, das erhaltene Produktgemisch säulenchromatographisch an Kieselgel gereinigt wird.
Dieses Reinigungsverfahren ist zur Herstellung von Mengen, die dem industriellen Bedarf entsprechen, im allgemeinen zu teuer und aufwendig und wird höchstens zur Reinigung von Endprodukten oder wertvollen Zwischenprodukten eingesetzt. Darüber hinaus werden zur Synthese von Oligosacchariden sehr häufig Schwermetallverbindungen eingesetzt. Ihr Einsatz für die Synthese von Stoffen mit pharmazeutischer Wirkung ist im Hinblick auf eine zukünftige Zulassung des Kohlenhydratrezeptorblockers als Arzneimittel sehr bedenklich.

Bei dem beschriebenen Verfahren wird das gewünschte Oligosaccharid erst nach dessen Aufbau über zahlreiche chemische und/oder chemoenzymatische Syntheseestufen mittels des Spacers an das bioabbaubare Polymer geknüpft. Diese Synthese ist wegen der dem Fachmann bekannten Problematik des Aufbaus von Oligosacchariden (Schutzgruppen, Anomerenbildung, schlechte Ausbeuten bei Glycosylierungsreaktionen, nicht stereoselektive Glycosylierung, zahlreiche Stufen) sehr langwierig und schwierig.

In Anbetracht der sehr hohen zu betreibenden Aufwands aufeinanderfolgender Reaktions- und Reinigungsschritte bei der chemischen und/oder chemoenzymatischen Synthese von Kohlenhydratbausteinen wurden in letzter Zeit auch einige Festphasensynthesen vorgeschlagen.

Im Gegensatz zu den etablierten Festphasensynthesen von Oligonucleotiden und Peptiden ist die chemische Synthese von Oligosacchariden an polymeren Festphasen durch die Vielzahl der Funktionalitäten und die Notwendigkeit der stereoselektiven Knüpfung der glycosidischen Bindung sehr schwierig. Danishefsky et al. (Science, 260, 1307 (1993)) verknüpfen 3,4-geschütztes Glycal über Silyether-Bindungen mit einem Polystyrol-Copolymer. Dieses wird als Epoxid aktiviert und kann mit weiteren Glycal-Acceptoren zum Oligosaccharid verknüpft werden. Douglas et al. (J. Am. Chem. Soc. 113, 5095 (1991) beschreiben die Synthese von Di- und Trisacchariden an einem PEG-gebundenen Glucosebaustein. Zehavi (J. Am. Chem. Soc. 95, 5673 (1973)) verwendet als polymere Festphase ein lichtempfindliches Styrol-Divinylbenzol- Copolymer. Das geschützte Oligosaccharid wird durch Bestrahlung vom Polymer abgespalten.

Nachteil der chemischen Festphasensynthese von Oligosacchariden sind:
- z. T. unvollständige Glycosylierungsreaktionen
- nur wenige Glycosylierungsbausteine sowohl auf der Donor- als auch der Acceptorseite sind geeignet.
- Notwendigkeit von der jeweiligen Reaktion angepaßten Schutzgruppen.

Diese Nachteile der chemischen Synthese von Oligosacchariden an polymeren Matrices werden durch die enzymatische Glycosylierung vermieden. Die Reaktionen verlaufen ohne Schutzgruppen absolut stereoselektiv und sind durch die Vielzahl der verfügbaren Glycosyltransferasen und Nucleotid-aktivierten Zucker als Glycosyldonoren sehr breit einsetzbar.
Bereits 1980 beschreiben Nunez und Barker (Biochemistry 19, 489 (1980) die enzymatische Galactosylierung von über einen Hexanolamin-Spacer an Agarose gebundenen N-Acetylglucosamin. Allerdings werden sehr große Enzymmengen an Galactosyltransferase eingesetzt. U. Zehavi beschreibt die enzymatische Galactosylierung mit Galactosyltransferase an lichtsensitiven sowohl wasserunlöslichen als auch wasserlöslichen Polymeren. Die Transferausbeuten sind mit <1 bis maximal 36 % sehr niedrig (U. Zehavi et al., Carbohydrate Res. 124, 23 (1983), U. Zehavi et al. Carbohydrate Res. 128, 160 (1984), U. Zehavi Reactive Polymers 6, 189 (1987), U. Zehavi et al. Glycoconjugate J. 7, 229 (1990), U. Zehavi Innovation Perspect. Solid Phase Synthesis Collect. Paper, Int. Symp. 1990, 389 bis 396). Das am Polymer erhaltene Disaccharid wird durch Lichteinwirkung oder über ein Enzym (U. Zehavi et al. Carbohydrate Res. 133, 339 (1984)) vom Polymer abgespalten.
Nishimura (Nishimura et al. Biochemical and Biophysical Research Comm. 199, 249-254 (1994))) beschreibt die enzymatische Herstellung eines wasserlöslichen Polyacrylamides mit 3'-Sialyl-N-acetyllactosamin-Seitenketten, wobei ein wasserlösliches, N-Acetylglucosamin tragendes Polyacrylamid stufenweise enzymatisch glycosyliert wird. Bei niedrigen Gesamtausbeuten werden jedoch geringe Belegungsdichten bei diesem Verfahren erreicht. Eine neuere Arbeit (Wong et al. J. Am. Chem. Soc. 116,1135 (1994)) beschreibt die enzymatische Synthese von Oligosacchariden an einem modifizierten Kieselgel. Bedingt durch die Unlöslichkeit des Kieselgels in den für die enzymatische Kohlenhydratsynthese benötigten wäßrigen Puffern und die geringe Belegungsdichte mit dem über ein Peptid verknüpften GlcNAc-Baustein werden nur geringe Glycosylierungsausbeuten in allen drei Reaktionsschritten erzielt. Nach enzymatischer Spaltung des Peptidankers erhält man daher Produktgemische mit nur 20 % des gewünschten Produkts und 45 % des eingesetzten Edukts.

In der WO 92/22661, der WO 92/22565 und der WO 92/22563 werden enzymatische Glycosylierungen mit einer Sialytransferase von an einem "nicht natürlichen Träger" (artificial carrier) gebundenen Disacchariden vorgeschlagen. Ein "nicht natürlicher Träger" ist in der Regel ein hoch- oder niedermolekularer Träger mit antigenen Eigenschaften, z.B. Rinderserumalbumin, KLH, HSA, Diphterie oder Tetanustoxin etc. oder ein fester Träger, der gegenüber physiologischen Systemen inert ist.

Ausgehend von dem genannten Stand der Technik ist es Aufgabe der vorliegenden Erfindung, ein Verfahren zur Herstellung des eingangs beschriebenen polyvalenten, physiologisch verträglichen und physiologisch abbaubaren Kohlenhydratrezeptorblockers bereitzustellen, welches Verfahren sich dadurch auszeichnet, daß der Aufbau des Kohlenhydratanteils des Rezeptorblockers durch enzymatische Glycosylierungsreaktionen in wäßrigen Puffersystemen und homogener Phase direkt am biodegradablen Polymer erfolgt, die Ausbeuten der Glycosylierungsreaktion gegenüber den Ausbeuten bekannter Verfahren wesentlich verbessert sind und in der Regel quantitativ verlaufen und die Belegungsdichten an Oligosaccharid auf dem Polymer deutlich erhöht sind, sowie eine Verwendung des erfindungsgemäß hergestellten Kohlenhydratrezeptorblockers zur Herstellung der freien Oligosaccharide vorzuschlagen.

Die Aufgabe wird erfindungsgemäß gelöst durch ein Verfahren zur Herstellung eines physiologisch verträglichen und physiologisch abbaubaren Kohlenhydratrezeptorblockers auf Polymerbasis bestehend aus
a) einer hydrophilen, biodegradablen Polymereinheit,
b) wenigstens einer Di- oder Oligosaccharideinheit und
c) wenigstens einem bifunktionellem Spacer, über welchen die Di- oder Oligosaccharideinheiten mit der Polymereinheit verknüpft sind,
dadurch gekennzeichnet, daß durch chemische Verknüpfung eines Mono- oder Oligosaccharids, des Spacers und des hydrophilen, biodegradablen Polymers zunächst ein Akzeptor hergestellt wird, wonach ein oder mehrere weitere Monosaccharidbausteine durch enzymatische Glycosylierung angefügt werden.

Die enzymatische Glycosylierungsreaktion verläuft stereoselektiv und mit überraschend hohen Ausbeuten direkt am Polymer und kann, da jeder Gycosylierungsschritt quantitativ verläuft, beliebig oft mit beliebigen Donoren wiederholt werden. Auf diesem Wege ist die Erzeugung polyvalenter Kohlenhydratverbindungen durch direkten Aufbau der Oligosaccharidstrukturen am Polymer im Gegensatz zum Stand der Technik, wo die Ausbeuten ering sind und daher Produktgemische bei weiteren Glycosylierungsschritten entstehen, sehr einfach und in sehr hohen Ausbeuten möglich. Ein weiterer Vorteil ist die einfache Isolierung und die Einheitlichkeit der polyvalenten Kohlenhydratverbindung.

Der Akzeptor für die enzymatische Glycosylierung wird durch Bildung einer kovalenten Bindung zwischen einem Mono- oder Oligosaccharid und dem bifunktionellem Spacer, anschließender kovalenten Verknüpfung des Mono- oder Oligosaccharid-Spacer-Komplexes mit dem Polymer hergestellt.

Die enzymatische Glycosylierung am Akzeptor erfolgt in homogener wäßriger Phase, vorzugsweise mittels nucleotidaktivierten Kohlenhydraten als Donoren und Glycosyltransferasen.

Das wäßrige Medium sollte aus einem Puffersystem bestehen, das an die jeweilige Glycosyltransferase angepaßt ist, vorzugsweise liegt das Puffersystem in einer Konzentration von 0,01 M bis 1 M vor und enthält vorteilhafterweise die zur Aktivierung der jeweiligen Glycosyltransferase notwendigen Kationen.

Der pH-Wert liegt zwischen 6,0 und 8,5, bevorzugt zwischen 6,0 und 8,5, ganz besonders bevorzugt zwischen 7,0 und 7,5.

Bei äquimolarem oder überschüssigem Zusatz des Donors sollte dem Reaktionsmedium alkalische Phosphatase zugegeben werden.

Dem Reaktionsgemisch werden 0,01 bis 10 Units der Glycosyltransferase zugegeben.

Die enzymatische Glycosylierung wird 1 bis 5 Tage bei 10 bis 40°C, bevorzugt bei 20 bis 37°C, noch bevorzugter bei 25 bis 37°C, durchgeführt.

Im folgenden wird die Erfindung detailliert erläutert:

### 1. SYNTHESE DES ACCEPTORS FÜR DIE ENZYMATISCHE GLYCOSYLIERUNGSREAKTION

Der Acceptor für die enzymatische Glycosylierungsreaktion besteht aus einem Mono- oder Oligosaccharid, das kovalent über einen Spacer an ein biodegradables hydrophiles Polymer verknüpft ist. Das Polymer kann mit einem Wirkungsverstärker versehen sein. Die Synthese des Acceptors erfolgt nach dem Fachmann bekannten Methoden.
Im folgenden werden die einzelnen Bausteine des Acceptors beschrieben.

### Biodegradables hydrophiles Polymer:

Definitionsgemäß besteht das Polymer aus wenigstens zwei gleichen oder verschiedenen Monomerbausteinen, die linear oder verzweigt miteinander verknüpft sind und eine Molekulargewichtsverteilung aufweisen können.

Das Polymer ist vorzugsweise eine Polyaminosäure als Polyamid oder -anhydrid verknüpft mit einem Molekulargewicht kleiner oder gleich 70 kD. Vorzugsweise hat das Polymer eine Mindestgröße von 2 kD, um im Vergleich zu niedermolekularen Trägern eine erhöhte Verweilzeit im Blut zu erzielen.

Für die Herstellung von Kohlenhydratrezeptorblockern auf Polymerbasis besonders bevorzugt geeignete Polyaminosäuren sind Polyaspartamide, Polysuccinimide, Polyglutamate und Polylysinfumaramide, wie z.B. Poly-α,β-(2-hydroxyethyl)-D,L-aspartamid, Poly-D,L-succinimid, Polyglutamat, Poly-L-Lysinmethyl-ester-fumaramid, sowie deren Copolymere.

Die Herstellung von biodegradablem, hydrophilen Polymer erfolgt nach dem Fachmann bekannten Verfahren. Diese sind z.B. beschrieben in: H.G. Elias, Makromoleküle, Bd. 1 und 2, Hüthig & Wepf Verlag, Basel, Schweiz, 1991/92 oder D. Braun, H. Cherdron, W. Kern, Praktikum der Makromolekularen Organischen Chemie, Hüthig Verlag 1979.

So wird z.B. Poly-D,L-Succinimid (PSI) entsprechend der Vorschrift von Neri et al., J. Med. Chem., 16, 893 (1973) durch Einwirkung von 85 %iger Phosphorsäure auf Asparaginsäure bei Temperaturen von 160°C - 180°C gewonnen. Durch polymeranaloge Umsetzung von PSI mit Hydroxyethylamin bei Raumtempertur oder leicht erhöhter Temperatur erhält man Poly-α,β-(2-hydroxyethyl)-D,L-aspartamid (PHEA) (Neri et al., ibid). Die Alkoholgruppen des PHEA lassen sich nach üblichen Verfahren verestern (US 5.041.291). Bei partieller Umsetzung von PSI mit Ethanolamin erhält man entsprechende Copolymere (US 5.229.469). Die basische Hydrolyse von PSI führt zur Polyasparaginsäure (analog Giammona et al., Chem. Pharm. Bull. 37(8), 2245 (1989).

Analog zur Reaktion mit Hydroxyethylamin kann PSI auch mit anderen Aminen umgesetzt werden (EP 0 548 794), wodurch sich zusätzliche funktionelle Gruppen einführen lassen, die als Wirkungsverstärker fungieren können.

Poly-L-Lysinmethylester-fumaramid, als ein weiteres Ausgangspolymer, wird durch Grenzphasenpolykondensation aus L-Lysinmethylester und Fumarsäuredichlorid hergestellt (US 4.834.248). Die Methylestergruppen können direkt oder nach partieller Hydrolyse und anschließender Aktivierung, z.B. als p-Nitrophenylester, mit den aminogruppenhaltigen Mono-, Di- und Oligosacchariden umgesetzt werden.

Analog, d.h. mittels p-Nitrophenylester, lassen sich polymere Kohlenhydratrezeptorblocker auf Basis von Polyglutarmaten herstellen (Polymersynthese analog: Anderson in "Macromolecules as Drugs and as Carriers for Biologically active Materials" (Ed: D.A. Tirell), NY Acad. Sci., NY, 1985, S. 67 - 75).

### Spacer:

Die kovalente Verknüpfung des Polymers mit dem Spacer bzw. mit einer Verbindung bestehend aus kovalent verküpftem Spacer und Kohlenhydrat sowie einer kovalenten Verbindung aus Polymer und Wirkungsverstärker mit dem Spacer bzw. mit einer Verbindung bestehend aus kovalent verknüpftem Spacer und Kohlenhydrat erfolgt durch Reaktion zwischen einer reaktiven Gruppe und einer aktivierten Gruppe. Dabei können sich sowohl die reaktive Gruppe am Ende des Spacers bzw. einer Verbindung bestehend aus kovalent verknüpftem Spacer und Kohlenhydrat und die aktivierte Gruppe auf Seiten des Polymers bzw. einer Verbindung bestehend aus kovalent verknüpftem Polymer und Wirkungsverstärker als auch die aktivierte Gruppe am Ende des Spacers bzw. einer Verbindung aus kovalent verknüpftem Spacer und Kohlenhydrat und die reaktive Gruppe auf Seiten des Polymers bzw. einer Verbindung bestehend aus kovalent verknüpftem Polymer und Wirkungsverstärker befinden. Die Reaktion zwischen reaktiven und aktivierten Gruppen erfolgen nach dem Fachmann bekannten Verfahren zur Alkylierung, Acylierung oder Addition an eine Doppelbindung. Diese Verfahren sind dem Fachmann aus der Literatur bekannt. (Larock, R.C. Comprehensive Organic Transformations, 1989, VCH Verlagsgesellschaft Weinheim).

Der Spacer hat vorzugsweise die Formel I

(Mono- oder Oligosaccharid)-O-[Q¹ -(CH₂)ₚ-Q²]ᵣ-(Polymereinheit) I,

worin bedeuten
- Q¹: -CH₂- oder 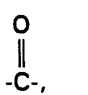
- Q²: -NH- oder 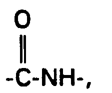
- p: eine ganze Zahl von 1 bis 6 und
- r: 1 oder 2

### Kohlenhydratanteil des Akzeptors

Der Kohlenhydratanteil des Akzeptors für die enzymatische Glycosylierungsreaktion kann aus natürlichen Quellen stammen oder chemisch, chemoenzymatisch oder enzymatisch hergestellt werden. Geeignete natürliche Quellen für Kohlenhydrate sind dem Fachmann bekannt und können in der biochemischen Literatur nachgelesen werden. dort werden ebenfalls etablierte, dem Fachmann bekannte Verfahren zur Aufreinigung von Oligosacchariden beschrieben.

Verfahren zur chemischen, enzymatischen oder chemoenzymatischen Synthese von Kohlenhydraten, die von Zelloberflächenrezeptoren erkannt werden, sind dem Fachmann aus der chemischen Literatur sowie aus Übersichtsartikeln bekannt. Für die chemische Synthese z.B. Carbohydrate Research, Elsevier Science Publishers B.U. Amsterdam; Journal of Carbohydrate Chemistry, Marcel Dekker Inc. New York; H. Paulsen, Angew. Chem. 94. 184 (1982) und 102, 851 (1990); R. R. Schmidt Angew. Chem. 98, 213 (1987); H. Kunz Angew. Chem. 98, 247 (1987). Für die enzymatische Synthese z.B. Carbohydrate Research, Elsevier Science Publishers B. U. Amsterdam; Journal of Carbohydrate Chemistry, Marcel Dekker Inc. New York; Bednarski u. Simon Enzymes in Carbohydrate Synthesis, ACS Symposium Series 466 (1991); K. G. I. Nilsson, Applied Biocatalysis 1991, 117; S. David et al. Adv. Carbohydr. Chem. Biochem. 49, 175 (1991); Y. Ichikawa et al. Anal. Biochem. 202, 215 (1992); D. G. Drueckhammer et al. Synthesis 1991, 499; E. J. Toone et al. Tetrahedron 45, 5365 (1989).

Die auf diesem Wege hergestellten Mono- oder Oligosaccharide können sowohl mit freiem reduzierenden Ende als auch in einer Spacer-verknüpften Form erhalten werden. Die Einführung des Spacers erfolgt nach dem Fachmann bekannten Verfahren zur chemischen oder enzymatischen Glycosylierung.

### 2. ENZYMATISCHE GLYCOSYLIERUNG

Im folgenden wird das erfindungsgemäße Verfahren zur Herstellung eines polymeren Kohlenhydratrezeptorblockers durch enzymatsiche Glycosylierung beschrieben:

Der nach 1. erhaltene Acceptor für die enzymatische Glycosylierungsreaktion besteht aus einem Mono- oder Oligosaccharid, das kovalent über einen Spacer an ein biodegradables hydrophiles Polymer verknüpft ist. Das Polymer kann mit einem Wirkungsverstärker versehen sein. Vorzugsweise erfolgt die enzymatische Glycosylierung des Akzeptors in homogener, wäßriger Phase. Vorzugsweise wurden nucleotidaktivierte Kohlenhydrate als Donoren und Glycosyltransferasen als Enzyme eingesetzt.

Der Acceptor wird in einem wäßrigen Puffersystem gelöst. Der Puffer ist der jeweiligen Glycosyltransferase angepaßt und kann z.B. aus 0,01M bis 1M Cacodylat, HEPES, PIPES, MOPS, Citrat, Hydrogencarbonat usw. bestehen. Er enthält die zur Aktivierung der jeweiligen Glycosyltransferase notwendigen Kationen, z.B. Mn.

Der pH-Wert wird ebenfalls der jeweiligen Glycosyltransferase angepaßt, er liegt zwischen 6,0 und 8,5, bevorzugt zwischen 6,5 und 7,8, ganz besonders bevorzugt zwischen 7,0 und 7,5.

Nach Lösen des Acceptors im wäßrigen Puffersystem wird der Donor zugegeben. Dieser ist ein Nukleotid-aktivierter Zucker oder ein Analogon eines Nukleotid-aktivierten Zuckers. Die Nukleotid-aktivierten Zucker sind teilweise käuflich, konnen aber auch nach dem Fachmann bekannten Methoden durch chemische oder enzymatische Synthesen hergestellt oder aus natürlichen Quellen isoliert werden. Dies gilt auch für die Analoga. Der Donor wird entweder im 1.1 bis 2fachen Überschuß zugegeben oder nach bekannten Verfahren in situ regeneriert (z.B. Y. Ichikawa et al. J. Am. Chem. Soc. 114, 9283 (1992), C. H. Wong et al. J. Org. Chem. 57, 4343 (1992), Y. Ichikawa et al. J. Am. Chem. Soc. 113, 6300 (1991), C. H. Wong et al. J. Org. Chem. 47,5416 (1982).

Bei der enzymatischen Galactosylierung wird in der Regel UDP-Galactose als Donor eingesetzt. Es kann aber auch von UDP-Glucose ausgegangen werden, die durch das Enzym UDP-Galactose-4-Epimerase enzymatisch in situ zu UDP-Galactose epimerisiert werden kann (J. Thiem et al. Angew. Chem. 102, 78 (1990)).

Wird der Donor bei der enzymatischen Glycosylierung äquimolar oder im Überschuß eingesetzt, ist es notwendig, das bei der Reaktion freiwerdenende UDP enzymatisch durch Zusatz alkalischer Phosphatase zu zersetzen, um eine Inhibition der Glycosyltransferase zu verhindern (C. Unverzagt et al. J. Am. Chem. Soc. 112, 9308 (1990)).

Nukleotid-aktivierte Zucker sind z.B. UDP-Glucose, UDP-Galactose, UDP-N-Acetyl-glucosamin, UDP-N-Acetylgalactosamin, UDP-Glucuronsäure, CMP-Neuraminsäure, GDP-Fucose, GDP-Mannose, dTDP-Glucose, dUDP-Galactose.
Dem Fachmann bekannte Verfahren zur Herstellung von Nucleotid-aktivierten Zuckern sind z.B.: M. Kittelmann et al. Annals of the New York Academy of Sciences Vol. 672 Enzyme Engineering, S. 444 bis 450 (1992), S. Makino et al. Tetrahedron Lett. 34, 2775 (1993), T. J. Martin et al. Tetrahedron Lett. 34, 1765 (1993), Europäische Patentanmeldung 0 524 143 Al; K. Ikeda, Carbohydrate Res. 224, 123 (1992), E. L. Kean Glycobiology 1, 441 (1991); Y. Ichikawa et al. J. Org. Chem. 57, 2943 (1992), K. Adelhorst et al. Carbohydrate Research 242, 69 (1993), R. R. Schmidt et al. Lieb. Ann. Chem. 1991. 121, R. Stiller et al. Lieb. Ann. Chem. 1992, 467; J. E. Heidlas et al. J. Org. Chem. 57, 146 (1992), J. E. Heidlas Acc. Chem. Res. 25, 307 (1992), E. S. Simon et al. J. Org. Chem. 55, 1834 (1990), C. H. Wong et al. J. Org. Chem. 57, 4343 (1992), J. E. Pallanca et al. J. Chem. Soc. Perkin Trans. 1 1993, 3017.

Zu dem im wäßrigen Puffersystem gelösten Acceptor und dem Nucleotid-aktivierten Zucker werden 0,01 bis 10 Units der Glycosyltransferase gegeben, die den jeweiligen Nucleotid-aktivierten Zucker auf den Acceptor übertragen kann.
Die Glycosyltransferasen sind teilweise käuflich, aus natürlichen Quellen isolierbar oder rekombinant verfügbar. Glycosyltransferasen, die für die enzymatische Glycosylierung im Sinne des erfindungsgemäßen Verfahrens verwendet werden können sind z.B.
β-1,4Galactosyltransferase [R. Barker et al. J. Biol. Chem. 247, 7135 (1972),C.H. Krezhorn et al. Eur. J. Biochem. 212, 113 (1993),
Gal-β-1-4-GlcNAc-α-2-6-Sialyltransferase [J.C. Paulson et al. J. Biol. Chem. 252, 2363 (1977), H. Higa et al. J. Biol. Chem. 260, 8838 (1985), J. Weinstein et al. J. Biol. Chem. 257, 13835 (1982)],
Gal-β-1-3GalNAc-α-2-3-Sialyltransferase [ W. Gillespie et al. J. Biol. Chem. 267, 21004 (1992)],
Gal-β-1-3(4)-GlcNAc-α-2-3-Sialyltransferase [J. Weinstein et al. J. Biol. Chem. 257, 13835 (1982), M. Nemansky et al. Glycoconjugate J. 10, 99 (1993)],
GalNAc-α-2-6-Sialytransferase [H.J: Gross et al. Biochemistry 28, 7386 (1989), N-Acetylglucosaminyltransferasen [R. Oehrlein et al. Carbohydrate Res. 244, 149 (1993), T. Szumilo et al. Biochemistry 26, 5498 (1987), O. Hindsgaul et al. J. Biol. Chem. 266, 17858 (1991), G. C. Look et al. J. Org. Chem. 58, 4326 (1993)],
α-1-3-Fucosyltransferase [B.W. Weston, J. Biol.Chem. 267, 4152 (1992)],
α-1-2-Fucosyltransferase [T.A. Beyer, J. Biol. CHem. 255, 5364 (1980)],
α-3/4-Fucosyltransferase [P.H. Johnson Glycoconjugate J. 9, 241 (1992)],
α-1-2-Mannosyltransferase [P. Wang, J. Org. Chem. 58, 3985 (1993)], Allgemein: T. A. Beyer et al. Advances in Enzymology Vol. 52, 23 bis 175 (1981), WO 93/13198

Die enzymatische Glycosylierung wird 1 bis 5 Tage bei 10 bis 40°C, vorzugsweise bei 20 bis 37°C, besonders vorzugsweise bei 25 bis 37°C durchgeführt.

Zur Aufarbeitung wird die Produktlösung nach Beendigung der Reaktion, erkennbar durch chromatographische Methoden (DC, HPLC), gegen bidest. Wasser dialysiert. Der Kohlenhydratrezeptorblocker kann anschließend durch chromatographische Methoden wie z.B. Gelchromatographie weiter gereinigt werden.

Das erfindungsgemäße Verfahren ist zur Herstellung von Kohlenhydratrezeptorblockern mit den folgenden Oligo- oder Disaccharideinheiten besonders geeignet:
Galβ1-4GlcNAc-;
Galβ1-3GlcNAc-,
SA*α*2-6Galβ1-4GlcNAc-;
SA*α* 2-3Galβ1-4GlcNAc-;
SAα2-3Galβ1-3GlcNAc-;
Galβ1-4<Fucα1-3)GlcNAc-;
Galβ1-3<Fucα1-3)GlcNAc-;
SAα2-3Galβ1-3(Fucα1-4)GlcNAc-;
SAα2-3Galβ1-4(Fucα1-3)GlcNAc-;

Sialyl-Lewis X, Sialyl-Lewis A, VIM-2 sowie die folgenden Blutgruppendeterminanten Lewis A, B, X, Y und A-Typ¹, A-Typ², B-Typ¹, B-Typ² und H-Typ¹, H-Typ² (R.U. Lemieux, Chem. Soc. Rev., 1978, S. 423 und 1989, S. 347) und fernder Sialyl-Lewix X, Sialyl-Lewis A oder VIM-2.

Die Formel von Sialyl-Lewis X lautet: NeuNAcα2-3Galβ1-4(Fucα1-3)GlcNAc und von Sialyl-Lewis A: NeuNAcα2-3Galβ1-3(Fucα1-4)GlcNAc. Die Formel von VIM-2 lautet: NeuNAcα2-3Galβ1-4GlcNAcβ1-3Galβ1-4(Fucα1-3)GlcNAc;

Im folgenden werden beispielhaft die Vorschriften für die Synthese beschrieben.

Umsetzung des Polymers mit dem Kohlenhydratanteil mit bifunktionellem Spacer einerseits und dem Wirkungsverstärker andererseits unter Ausbildung kovalenter Bindungen:

### Beispiel 1: 1-(6-Aminohexyl)-2-deoxy-2-acetamido-β-D-Glucopyranosid

2-Amino-2-deoxyglucose-Hydrochlorid wird entsprechend der Vorschrift von R. U. Lemieux et al. (ACS Symp. Ser. 39, 90 (1976) durch Umsetzung mit Phthalsäureanhydrid und anschließender Reaktion mit Acetanhydrid/Pyridin in 1,3,4, 6-Tetraacetyl-2-N-acetyl-2-deoxyglucose überführt.
Durch Behandlung mit Zinntetrachlorid/Thiophenol entsteht nach Nicolaou et al. (J. Am. Chem. Soc. 112, 3695 (1990)) das entsprechende 1-Thiophenylderivat. Dieses wird nach der Vorschrift von B. A. Silwanis et al. (J. Carbohydr. Chem. 10, 1067 (1991)) mit 6-(N-Benzyloxycarbonyl)-aminohexanol umgesetzt. Analog zu der Vorschrift von K. C. Nicolaou et al. (J. Am. Chem. Soc. 114, 3127 (1992)) erfolgt die Spaltung der Acetyl- und der Phthaloylschutzgruppen mit Hydrazinhydrat. Vor Abspaltung der Benzylschutzgruppen (H2/Pd(OH)2, MeOH) wird die freie Aminogruppe selektiv in Gegenwart der freien Hydroxylgruppen mit überschüssigem Acetanhydrid acetyliert. Man erhält 1-(6-Aminohexyl)-2-deoxy-2-acetamido-β-D-Glucopyranosid

### Beispiel 2: Poly-D,L-succinimid-co-α,β-D,L-aspartamido-6-hexyl-2-deoxy-2-acet amido-β-D-Glucopyranose (Poly-D,L-succinimid-co-α,β-D,L-aspartamido-C₆-GlcNAc)

PSI (500 mg, MG 24 000) wird in 2 mL DMF gelöst und mit 225 mg (0,71 mmol) 1-(6-Aminohexyl)-2-deoxy-2-acetamido-β-D-Glucopyranosid (GlcNAc-C₆-NH₂) in 2,5 ml DMF versetzt. Der Ansatz wird 5,5 Stunden bei Raumtemperatur unter N₂ gerührt. Danach wird mit 40 ml 1-Butanol gefällt und das erhaltene Polymer mit Methanol gewaschen. Nach einer zweiten Fällung aus DMF in 1-Butanol wird erneut mit Methanol gewaschen und anschließend im Ölpumpenvakuum getrocknet.
Ausbeute: 490 mg
Substitutionsgrad laut NMR: 12,5 %

### Beispiel 3: Poly-D,L-succinimid-co-α,β-D,L-aspartamido-6-hexyl-2-deoxy-2-acet amido-β-D-Glucopyranose (Poly-D,L-succinimid-co-α,β-D,L-aspartamido-C₆-GlcNAc)

Analog zu Beispiel 2 werden 320 mg PSI (MG 24 000) mit 300 mg 1-(6-Aminohexyl)-2-deoxy-2-acetamido-β-D-Glucopyranosid (GlcNAc-C₆-NH₂) umgesetzt und durch zweimalige Fällung aus DMF mit 1-Butanol aufgearbeitet.
Ausbeute: 383 mg
Substitutionsgrad laut NMR: 18,5 %

### Beispiel 4: Poly-D,L-succinimid-co-α,β-D,L-aspartamido-6-hexyl-2-deoxy-2-acet amido-β-D-Glucopyranose (Poly-D,L-succinimid-co-α,β-D,L-aspartamido-C₆-GlcNAc)

Analog zu Beispiel 2 werden 480 mg PSI (MG 9 600) mit 210 mg 1-(6-Aminohexyl)-2-deoxy-2-acetamido-β-D-Glucopyranosid (GlcNAc-C₆-NH₂) umgesetzt und durch zweimalige Fällung aus DMF mit 1-Butanol aufgearbeitet.
Ausbeute: 485 mg
Substitutionsgrad laut NMR: 12,5 %

### Beispiel 5: Poly-D,L-succinimid-co-α,β-D,L-aspartamido-6-hexyl-2-deoxy-2-acet amido-β-D-Glucopyranose (Poly-D,L-succinimid-co-α,β-D,L-aspartamido-C₆-GlcNAc)

Analog zu Beispiel 2 werden 300 mg PSI (MG 9 600) mit 280 mg 1-(6-Aminohexyl)-2-deoxy-2-acetamido-β-D-Glucopyranosid (GlcNAc-C₆-NH₂) umgesetzt und durch zweimalige Fällung aus DMF mit 1-Butanol aufgearbeitet.
Ausbeute: 331 mg
Substitutionsgrad laut NMR: 18 %

### Beispiel 6: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 2 (200 mg) wird in 2 ml DMF gelöst und mit frisch destilliertem Hydroxyethylamin (81 mg) versetzt. Nach 16 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 180 mg
Substitutionsgrad laut NMR: 87,5 % HEA, 12,5 % GlcNAc-C₆-NH₂

### Beispiel 7: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 3 (100 mg) wird in 2 ml DMF gelöst und mit frisch destilliertem Hydroxyethylamin (41 mg) versetzt. Nach 16 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 112 mg
Substitutionsgrad laut NMR: 81,5 % HEA, 18,5 % GlcNAc-C₆-NH₂

### Beispiel 8: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 4 (150 mg) wird in 2 ml DMF gelöst und mit frisch destilliertem Hydroxyethylamin (61 mg) versetzt. Nach 16 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 147 mg
Substitutionsgrad laut NMR: 87,5 % HEA, 12,5 % GlcNAc-C₆-NH₂

### Beispiel 9: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 5 (150 mg) wird in 2 ml DMF gelöst und mit frisch destilliertem Hydroxyethylamin (61 mg) versetzt. Nach 16 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 127 mg
Substitutionsgrad laut NMR: 82 % HEA, 18 % GlcNAc-C₆-NH₂

### Beispiel 10: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 2 (170 mg) wird in 2 ml DMF gelöst und mit frisch destilliertem Hydroxyethylamin (43 mg) versetzt. Nach 4 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 160 mg
Substitutionsgrad laut NMR: 61,5 % HEA, 12,5 % GlcNAc-C₆-NH₂

### Beispiel 11: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 3 (120 mg) wird in 2 ml DMF gelöst und mit frisch destilliertem Hydroxyethylamin (30 mg) versetzt. Nach 4 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 126 mg
Substitutionsgrad laut NMR: 45,5 % HEA, 18,5 % GlcNAc-C₆-NH₂

### Beispiel 12: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 4 (150 mg) wird in 2 ml DMF gelöst und mit frisch destilliertem Hydroxyethylamin (38 mg) versetzt. Nach 4 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 146 mg
Substitutionsgrad laut NMR: 56 % HEA, 12,5 % GlcNAc-C₆-NH₂

### Beispiel 13: Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

PSI-co-aspartamido-C₆-GlcNAc aus Beispiel 5 (150 mg) wird in 2 ml DMF gelöst und mit frisch destilliertem Hydroxyethylamin (38 mg) versetzt. Nach 4 Stunden Rühren unter N₂ bei Raumtemperatur wird mit 1-Butanol gefällt. Das Polymer wird mit Methanol gewaschen, in H₂O gelöst und gefriergetrocknet.
Ausbeute: 143 mg
Substitutionsgrad laut NMR: 50 % HEA, 12,5 % GlcNAc-C₆-NH₂

### Beispiel 14: Poly-D,L-succinimid-α,β-(5-Carboxypentyl)-D,L-aspartamid

500 mg PSI (MG 9 600) werden in 2 ml DMF gelöst und mit 1,38 g 6-Aminohexansäure, gelöst in 8 ml Formamid, und 1 ml Triethylamin versetzt. Es wird 13 Stunden bei 45°C gerührt und anschließend mit 1-Butanol gefällt. Nach Waschen des Polymers mit Methanol wird in H20 aufgenommen und gefriergetrocknet.
Ausbeute: 350 mg
Substitutionsgrad laut NMR: 8 % Aminohexansäure

### Beispiel 15: Poly-D,L-succinimid-co-α,β-(5-Carboxypentyl)-D,L-aspartamid

500 mg PSI (MG 24 000) werden in 2 ml DMF gelöst und mit 690 mg 6-Aminohexansäure, gelöst in 4 ml Formamid, und 1 ml Triethylamin versetzt. Es wird 3 d bei Raumtemperatur und 5 Stunden bei 45°C gerührt und anschließend mit 1-Butanol gefällt. Nach Waschen des Polymers mit Methanol wird in H₂O aufgenommen und gefriergetrocknet.
Ausbeute: 470 mg
Substitutionsgrad laut NMR: 12,5 % Aminohexansäure

### Beispiel 16: Poly-D,L-succinimid-co-α,β-(5-Carboxypentyl)-D,L-aspartamid-co-α, β-D,L-aspartamido-6-hexyl-2-deoxy-2-acetamido-β-D-Glucopyranose (PCPA-co-aspartamido-C₆-GlcNAc)

100 mg PCPA aus Beispiel 14 werden in 2 ml H₂O gelöst und mit 20 mg 1.(6-Aminohexyl)-2-deoxy-2-acetamido-β-D-Glucopyranosid (GlcNAc-C₆-NH₂) versetzt. Während 36 Stunden werden 4 mal je 20 mg 1 Ethyl-3-(3-Dimethylaminopropyl)- carbodiimid-Hydrochlorid (EDC) zugegeben. Nach Dialyse und Gefriertrocknung verbleibt das Produkt.
Ausbeute: 112 mg
Substitutionsgrad laut NMR: 8 % GlcNAc-O(CH₂)₆NH₂

### Beispiel 17: Poly-D,L-succinimid-co-α,β-(5-Carboxypentyl)-D,L-aspartamid-co-α, β-D,L-aspartamido-6-hexyl-2-deoxy-2-acetamido-β-D Glucopyranose (PCPA-co-aspartamido-C₆-GlcNAc)

100 mg PCPA aus Beispiel 15 werden in 2 ml H₂O gelöst und mit 20 mg 1-(6-Aminohexyl)-2-deoxy-2-acetamido-β-D-Glucopyranosid (GlcNAc-C₆-NH₂) versetzt. Während 36 Stunden werden 4 mal je 20 mg 1-Ethyl-3-(3-Dimethylaminopropyl)- carbodiimid-Hydrochlorid (EDC) zugegeben. Nach Dialyse und Gefriertrocknung verbleibt das Produkt.
Ausbeute: 137 mg
Substitutionsgrad laut NMR: 12,5 % GlcNAc-O(CH₂)₆NH₂

### Enzymatische Galactosylierung

### Beispiel 18: Enzymatische Galactosylierung von Poly-D,L-succinimid-co-α,β-aspartamido-C₆-GlcNAc

50 mg des Polymers aus Beispiel 2 werden in 10 ml 0,05M HEPES-Puffer pH 7,5 gelöst und mit 2 mg MnCl₂ und 55 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 8 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 74 mg
Substitutionsgrad LacNAc laut NMR: 12,5 %

### Beispiel 19: Enzymatische Galactosylierung von Poly-D,L-succinimid-co-α,β-aspartamido-C₆-GlcNAc

50 mg des Polymers aus Beispiel 3 werden in 10 ml 0,05M HEPES-Puffer pH 7,5 gelöst und mit 2 mg MnCl₂ und 55 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 8 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 76 mg
Substitutionsgrad LacNAc laut NMR: 18,5 %

### Beispiel 20: Enzymatische Galactosylierung von Poly-D,L-succinimid-co-α,β-aspartamido-C₆-GlcNAc

50 mg des Polymers aus Beispiel 4 werden in 10 ml 0,05M HEPES-Puffer pH 7,5 gelöst und mit 2 mg MnCl₂ und 55 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 8 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 77 mg
Substitutionsgrad LacNAc laut NMR: 12,5 %

### Beispiel 21: Enzymatische Galactosylierung von Poly-D,L-succinimid-co-α,β-aspartamido-C₆-GlcNAc

50 mg des Polymers aus Beispiel 5 werden in 10 ml 0,05M HEPES-Puffer pH 7,5 gelöst und mit 2 mg MnCl₂ und 55 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 8 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 78 mg
Substitutionsgrad LacNAc laut NMR: 18 %

### Beispiel 22: Enzymatische Galactosylierung von Poly-α,β-(2-hydroxyethyl)-D,L-aspartamid-co-a,b-D,L-aspartamido-6-hexyl-2-deoxy-2-acetamido-β-D-Glucopyranose (PHEA-co-aspartamido-C₆-GlcNAc)

50 mg des Polymers aus Beispiel 6 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 70,6 mg
Substitutionsgrad LacNAc laut NMR: 12,5 %

### Beispiel 23: Enzymatische Galactosylierung von Poly-α,β-(2-hydroxyethyl)-D,L-aspartamid-co-α,β-D,L-aspartamido-6-hexyl-2-deoxy-2-acetamido-β-D-Glucopyranose (PHEA-co-aspartamido-C₆-GlcNAc)

50 mg des Polymers aus Beispiel 7 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 69 mg
Substitutionsgrad LacNAc laut NMR: 18,5 %

### Beispiel 24: Enzymatische Galactosylierung von Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

50 mg des Polymers aus Beispiel 8 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 55 mg
Substitutionsgrad LacNAc laut NMR: 12,5 %

### Beispiel 25: Enzymatische Galactosylierung von Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

50 mg des Polymers aus Beispiel 9 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 55 mg
Substitutionsgrad LacNAc laut NMR: 18 %

### Beispiel 26: Enzymatische Galactosylierung von Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

50 mg des Polymers aus Beispiel 10 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 54 mg
Substitutionsgrad LacNAc laut NMR: 12,5 %

### Beispiel 27: Enzymatische Galactosylierung von Poly-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

50 mg des Polymers aus Beispiel 11 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 71 mg
Substitutionsgrad LacNAc laut NMR: 18,5 %

### Beispiel 28: Enzymatische Galactosylierung von Poly-α,β-(2-hydroxyethyl )-D,L-aspartamid-co-α,β-D,L-aspartamido-6 -hexyl-2-deoxy-2-acetamido-β-D-Glucopyranose (PHEA-co-aspartamido-C₆-GlcNAc)

50 mg des Polymers aus Beispiel 12 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 66 mg
Substitutionsgrad LacNAc laut NMR: 12,5 %

### Beispiel 29: Enzymatische Galactosylierung von PoIy-α,β-(2-hydroxyethyl)-D,L-aspartamid-co-α,β-D,L-aspartamido-6 -hexyl-2-deoxy-2-acetamido-β-D-Glucopyranose (PHEA-co-aspartamido-C₆-GlcNAc)

50 mg des Polymers aus Beispiel 13 werden in 10 ml 0,05M HEPES-Puffer pH 7,5 gelöst und mit 2 mg MnCl₂ und 40 mg UDP-Glucose sowie 1 mg Lactalbumin versetzt. Nach Zugabe von 2 U UDP-Galactose-4-Epimerase, 2 U Galactosyltransferase und 40 U alkalischer Phosphatase (aus Kälberdarm) wird 7 Tage bei 25°C inkubiert. Nach Dialyse gegen Wasser wird gefriergetrocknet und anschließend über Sephadex G 10 chromatographiert. Nach nochmaliger Gefriertrocknung verbleibt das Produkt.
Ausbeute: 64 mg
Substitutionsgrad LacNAc laut NMR: 18 %

### Enzymatische Sialylierung

### Beispiel 30: Enzymatische Sialylierung von Poly-D,L-succinimid-co-α,β-aspartamido-C₆-LacNAc

35 mg des Polymers aus Beispiel 18 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 36,6 mg
Substitutionsgrad 2,6-Sialyl-LacNAc: 12,5 % (0,65 µmol/mg Polymer)

### Beispiel 31: Enzymatische Sialylierung von Poly-D,L-succinimid-co-α,β-aspartamido-C₆-LacNAc

30 mg des Polymers aus Beispiel 19 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 32 mg
Substitutionsgrad 2,6-Sialyl-LacNAc: 18,5 % (0,77 µmol/mg Polymer)

### Beispiel 32: Enzymatische Sialylierung von Poly-D,L-succinimid-co-α,β-aspartamido-C₆-LacNAc

30 mg des Polymers aus Beispiel 20 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 21 mg
Substitutionsgrad 2,6-Sialyl-LacNAc: 12,5 % (0,65 µmol/mg Polymer)

### Beispiel 33: Enzymatische Sialylierung von poly-D,L-succinimid-co-α,β-aspartamido-C₆-LacNAc

35 mg des Polymers aus Beispiel 21 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 38 mg
Substitutionsgrad 2,6-Sialyl-LacNAc: 18 % (0,76 µmol/mg Polymer)

### Beispiel 34: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 22 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 29 mg
Substitutionsgrad 2,6-Sialyl-LacNAc: 12,5 % (0,51 µmol/mg Polymer)

### Beispiel 35: Enzymatische Sialylierung von PoIy-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 23 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 29 mg
Substitutionsgrad 2,6-Sialyl-LacNAc: 18,5 % (O,64 µmol/mg Polymer)

### Beispiel 36: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 24 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 34 mg
Substitutionsgrad 2,6-Sialyl-LacNAc: 12,5 % (0,51 µmol/mg Polymer)

### Beispiel 37: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

25 mg des Polymers aus Beispiel 25 werden in 2 ml 0,05M Natrium-Cacodylat-Puffer pH 7,8 gelöst und mit 1,5 mg Rinderserumalbumin, 2 mg MnCl₂ und 5 rng CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 25 mg
Substitutionsgrad 2,6-Sialyl-LacNAc: 18 % (0,63 µmol/mg Polymer)

### Beispiel 38: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 26 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 28 mg
Substitutionsgrad 2,6-Sialyl-LacNAc: 12,5 % (0,54 µmol/mg Polymer)

### Beispiel 39: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 27 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 33 mg
Substitutionsgrad 2,6-Sialyl-LacNAc: 18,5 % (O,69 µmol/mg Polymer)

### Beispiel 40: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 28 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 31 mg
Substitutionsgrad 2,6-Sialyl-LacNAc: 12,5 % (0,55 µmol/mg Polymer)

### Beispiel 41: Enzymatische Sialylierung von Poly-α,β-(2-hydroxyethyl)-D,L-aspartamid-co-α,β-D,L-aspartamido-6 -hexyl-O-(β-D-Galactopyranosyl)-(1-4)-2-deoxy-2-acetamido-β-D-Glu copyranose (PHEA-co-aspartamido-C₆-LacNAc)

35 mg des Polymers aus Beispiel 29 werden in 2 ml 0,05M Natrium-Cacodylat-Puffer pH 7,8 gelöst und mit 1,5 mg Rinderserumalbumin, 2 mg MnCl₂ und 5 mg CMP-Neuraminsäure versetzt. Nach Zugabe von 20 mU 2-6-Sialyltransferase und 20 U alkalischer Phosphatase wird 8 Tage bei 25°C inkubiert. Nach Dialyse gegen Wasser wird gefriergetrocknet und anschließend über Biogel P2 chromatographiert. Nach nochmaliger Gefriertrocknung verbleibt das Produkt.
Ausbeute: 15 mg
Substitutionsgrad 2,6-Sialyl-LacNAc: 18 % (0,68 µmol/mg Polymer)

### Beispiel 42

### A: PRIMÄRASSAYS ZUR UNTERSUCHUNG DER WIRKUNG VON POLYMEREN KOHLENHYDRATREZEPTORBLOCKERN AUF DIE ZELLANHEFTUNG AN REKOMBINANTE LÖSLICHE SELEKTIN-FUSIONSPROTEINE

Mit diesem Assay wird die Wirkung von polymergebundenen Kohlenhydratbausteinen auf die Zellanheftung von promyelozytischen Zellen mittels Selektinen nachgewiesen:

Um die Wirksamkeit von polymergebundenen Kohlenhydratbausteinen auf die Interaktion zwischen den E- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### A1. HERSTELLUNG VON L-SELEKTIN-IGG1

Zur Herstellung von löslichem L-Selektion-lgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F.M., Brent, R., Kingston, R.E., Moore, D.D., Seidman, J.G., Struhl, K. und Smith, J.A. 1990. Current Protocols in Molecular Biology, John Wiley, New York.). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben.

Walz, G., Aruffo, A., Kolanus, W., Bevilacqua, M. und Seed, B. 1990. Recognition by ELAM-1 of the sialyl-Lex determinant on myeloid and tumor cells. Science 250, 1132-1135.

### A2. HERSTELLUNG VON P-SELEKTIN-IGG1

Zur Herstellung des löslichen P-Selektin-lgG1 Fusionsproteins wird das von Aruffo et al., 1991 publizierte genetische Konstrukt "CD62Rg" verwendet. Die weitere Vorgehensweise entspricht der unter Al dargestellten Herstellung von L-Selektin-lgG1 .

Aruffo, A., Kolanus, W., Walz, G., Fredman, P. und Seed, B. 1991. CD62/P-Selectin recognition of myeloid and tumor cell sulfatides. Cell 67, 35-44.

### A3. HERSTELLUNG VON CD4-IGG1

Zur Herstellung des löslichen CD4-lgG1 Fusionsproteins wird das von Zettlemeissl et al., 1990 publizierte genetische Konstrukt "CD4:lgG1 hinge" verwendet. Die weitere Vorgehensweise entspricht der unter A1 dargestellten Herstellung von L-Selektin-lgG1.

Zettelmeissl, G., Gregersen, J.-P.-, Duport, J.M., Mehdi, S., Reiner, G. und Seed, B. 1990. Expression and characterization of human CD4: Immunoglobulin Fusion Proteins. DNA and Cell Biology 9, 347-353.

### A4. DURCHFÜHRUNG DES HL60 ZELLADHÄSIONSASSAYS AUF REKOMBINANTEN, LÖSLICHEN ADHÄSIONSMOLEKÜLEN

1. 96er Mikrotitertestplatten (Nunc Maxisorb) werden mit 100 µl eines in 50 mM Tris pH 9,5 verdünnten (1+ IOO) Ziege anti human lgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindunspuffer gegeben. Der Bindungspuffer hat die Zusammensetzung: 50 mM Hepes, pH 7,5; 100 mM NaCl; 1 mg/ml BSA; 2 mM MgCl2; 1mM CaCl2; 3 mM MnCl2; 0,02 % Natriumazid; 0,2 mM PMSF. Dazu werden 5 µl der Testsubstanz pipettiert, durch Schwenken der Platte vermischt und 10 Min. bei Raumtempeartur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl2; 1 mM CaCl2; 3 mM MnCl2; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

IC⁵⁰ von HL60 Zellanheftung an E-Selektin-lgG:

| | |
|---|---|
| Verbindung aus Bsp. 30 | 60 µM |
| 31 | 60 µM |
| 32 | 100 µM |
| 33 | 60 µM |
| 34 | 150 µM |

Vergleichswert:
IC⁵⁰ HL60 Zellanheftung an E-Selektin-IgG:
Sialyl-Le^{x}-O(CH₂)₆NH₂: 1 mM
(vgl.EPA 93 119 098.7)

### B. SEKUNDÄRASSAY ZUR UNTERSUCHUNG DER WIRKUNG VON POLYMEREN KOHLENHYDRATREZEPTORBLOCKERN AUF DIE ZELLANHEFTUNG AN STIMULIERTE HUMANE ENDOTHELZELLEN

Der Test der Wirksamkeit von polymeren Kohlenhydratrezeptorblockern auf die Zelladhäsion an rekombinante, lösliche Fusionsproteine ist ein hochspezifischer Assay, der auf der Interaktion einer Art von Adhäsionsmolekülen mit den entsprechenden Liganden beruht. Um die in vivo Situation von Zell-Zell Interaktionen zu simulieren, verwenden wir einen Assay, bei dem HL60-Zellen an stimulierte, humane Nabeschnurendothelzellen adhärieren.

### 1. Gewinnung der humanen Nabelschnurendothelzellen (HUVEC).

Nabelschnüre werden nach der Geburt in PBS mit 100.000 IU/L Penicillin, 100 mg/L Streptomycin, 50 mg/L Gentamycin, 50.000 IU/L Mycostatin bei +4°C bis zur Weiterverarbeitung aufgehoben.
Die längsten unverletzten Stücke werden aus der Nabeschnur mit einem Skalpell herausgeschnitten und auf frische Aluminiumfolie gelegt. Ein Ende der Nabelschnur wird mit einer Klemme verschlossen. Am anderen Ende inseriert man einen passenden Schlauch in die Nabelschnurvene und fixiert diesen durch Umbinden des Nabelschnurendes.

Die Vene wird durch das Schlauchstück mit Collagenaselösung gefüllt (50 mg Collagenase /100 ml 25 mM Hepes, pH 7,0) und 15 Min. bei 37°C inkubiert.

Um die Zellausbeute zu erhöhen, wird die Nabelschnur nach der Inkubation leicht massiert, um die noch anhängenden Endothelzellen abzulösen.

Anschließend läßt man die Zellsuspension aus der Vene in ein Kulturröhrchen mit Zellkulturmedium und 10 % Foetalem Kälberserum ausfließen. Die Vene wird mit PBS gewaschen, um die zurückgebliebenen Zellen zu erhalten.

Die Zellsuspension wird 5 Min. bei 500 g zentrifugiert; das Zellpellet anschließend in 4 ml Kulturmedium resuspendiert und die Zellen ausplattiert. Nach 3-4 Tagen sind die Zellen konfluent gewachsen und können passagiert werden.

Zur Überprüfung der Reinheit der Endothelzellkultur, wird die Kultur mit einem Antikörper gegen Faktor VIII für die Immunfluoreszenz gefärbt. Lediglich Endothelzellen, nicht jedoch kontaminierende Fibroblasten zeigen eine positive Reaktion.

### 2. Durchführung des Assays

20.000 Endothelzellen werden pro Näpfchen einer 96er Mikrotiterplatte ausplattiert und 24 Std. bei 37°C inkubiert. Die Endothelzellen dürfen dazu nicht öfter als 5-6 mal passagiert worden sein. Vier Std. vor dem Assay werden die Endothelzellen durch Zugabe von II-1 (finale Konzentration: 15 U/ml) stimuliert. Nach Entfernung des Kulturmediums werden die Zellen einmal mir RPMI-Medium ohne Serum gewaschen. Nach Entfernung und erneutem Pipettieren von 20 µl RPMI-Medium, erfolgt die Zugabe von Testsubstanzen.

3. Die weiteren Schritte des Assays, Markierung der HL60 Zellen, Aufbringen der HL60 Zellen, werden wie unter A4. Punkte 5. - 7. durchgeführt.

### C. SEKUNDÄRASSAY ZUR UNTERSUCHUNG DER WIRKUNG VON POI YME-REN KOHLENHYDRATREZEPTORBLOCKERN AUF DIE ZELLANHEFTUNG AN GEFRIERSCHNITTE VON LYMPHATISCHEM GEWEBE

In vitro läßt sich untersuchen, in welchem Maße Leukozyten an Endothelzellen auf Gefrierschnitten lymphatischer Gewebe binden. Diese Zell-Zell-Interaktionen beruhen auf der Interaktion zwischen Adhäsionsmolekülen auf der Oberfläche der gefriergeschnittenen Endothelzellen und den entsprechenden Liganden auf der Oberfläche von Leukozyten. Stellvertretend für primäre Leukozyten lassen sich HL60-Zellen verwenden, deren Oberflächen-Liganden in der wissenschaftlichen Literatur gut beschrieben sind. Ob eine Anheftung von HL60-Zellen auf Lymphknoten-Gefrierschnitte stattfindet, läßt sich an der Zahl gebundener HL60-Zellen bestimmen.
1. Axilläre, cervicale oder mesenteriale Lymphknoten werden aus frisch getöteten Ratten präpariert und in flüssigem Stickstoff schnell eingefroren.
2. Von den gefrorenen Lymphknoten werden 10 µm dicke Cryostat-Schnitte angefertigt, auf runde Deckgläser (Durchmesser 18 mm) übertragen und 2 Std. bei Raumtemperatur getrocknet.
3. Auf die Schnitte werden 20 µl Bindungspuffer pipettiert. Die Testsubstanzen werden hinzugefügt und 10 Min. bei Raumtemperatur inkubiert. HL60-Zellen werden wie unter A4. Punkt 5. markiert. 200.000 markierte HL60-Zellen in 100 µl Bindungspuffer werden pro Deckglas hinzugefügt und 10 Min. stehen gelassen. Die sedimentierenden Zellen gelangen dabei auf Endothelzellen, an die sie teilweise anheften.
4. Im 45°-Winkel werden die Deckgläser in Stoppuffer getaucht um die nich tangehefteten Zellen abzuspülen. Anschließend werden die Deckgläser in 4 % Formaldehyd in PBS für 10 Min. bei Raumtemperatur fixiert.
5. Im Immunfluoreszenzmikroskop (FITC-Anregung) werden Querschnitte von lymphatischen Blutgefäßen photografisch dokumentiert. Die angehefteten HL60-Zellen heben sich deutlich vom nicht gefärbten Hintergrund ab. Das Ergebnis wird als gebundene HL60-Zellen pro Flächeneinheit Endothel ausgedrückt.

### D. TERTIÄRASSAY ZUR UNTERSUCHUNG DER WIRKUNG VON POLYMEREN KOHLENHYDRATREZEPTORBOCKERN AUF DIE LEUKOZYTEN-ADHÄSION IN DER RATTE IN VIVO

Das nachfolgend aufgeführte Verfahren ist in der Lage, die Wirksamkeit von Substanzen in vivo festzustellen, die die Anheftung von Leukozyten an die Gefäßinnenwände hemmen.

Es ist bekannt, daß einige der zirkulierenden weißen Blutzellen die Tendenz besitzen, an den Innenwänden der Blutgefäße anzuheften. Diese Tendenz verstärkt sich erheblich bei Entzündungsvorgängen. Leukozyten stoßen normalerweise ständig gegen die Blutgefäßwände, diese Kollision ist jedoch elastisch, so daß die Zellen gewissermaßen zurückprallen und in die Zirkulation zurückgelangen. Bei Entzündungsvorgängen führen biochemische Veränderungen sowohl in den Leukozyten, aber auch in den die Blutgefäße auskleidenden Endothelzellen zu Veränderungen der Oberflächeneigenschaften beider Zellarten. Die Zellen verhalten sich adhäsiver. Diese Adhäsivität drückt sich zunächst in einer Tendenz der Leukozyten aus, nach Kollision mit dem Endothel auf den Endothelzellen zu rollen. Dieses Rollen der Leukozyten auf dem Endothel induziert weitere biochemische Reaktionen auf beiden Bindungspartnern in deren Folge die Zellanheftung verstärkt wird. Durch diese weitere Verstärkung der Adhäsivität verlangsamt sich das Rollen der Leukozyten, bis diese fest auf dem Endothel anhaften. Dem festen Anhaften folgt als weiterer Schritt das Auswandern der Leukozyten aus dem Blutgefäß.

Das Rollen der Leukozyten, die feste Anheftung und das Auswandern aus den Blutgefäßen läßt sich mit Leukozyten-stimulierenden Faktoren wie FMLP (f-Met-Leu-Phe), LPS (Lipopolysacchariden) oder TNF (tumor necrosis factor) induzieren. Eine mikroskopische Dokumentation dieser Vorgänge ist am präparierten Mesenterialgewebe, z.B. der Ratte, möglich. In die Blutbahn inkizierte Substanzen lassen sich daher daraufhin untersuchen, ob sie in der Lage sind, die induzierten Leukozyten-Adhäsionen zu beeinflussen.

Zur praktischen Durchführung dieser Untersuchung werden Ratten mit anaesthetisiert. Die Bauchhöhe wird eröffnet und ein Abschnitt des Dünndarms herausgezogen. Der Dünndarmabschnitt wird auf einem heizbaren Mikroskopiertisch ständig feucht gehalten. Zur mikroskopischen Betrachtung wird ein Bereich von Mesenterialgewebe mit Paraffinöl abgedeckt. Zur Kontrolle werden in diesem Bereich alle 5 Min. über einen Zeitraum von 30 Min. alle anhaftenden - nicht stimulierten - Leukozyten gezählt. Parallel dazu werden Blutdruck, Körpertemperatur und Fließgeschwindigkeit des Blutes aufgezeichnet. Die Testsubstanz wird durch kontinuerliche venöse Infusion während des gesamten Tests appliziert. Nach Aufbringen von Leukozyten-Stimulantien, die auf das Mesenterialgewebe getropft werden, werden die anheftenden Leukozyten alle 5 Min. über einen Zeitraum von 90 Min. gezählt.

Die Untersuchung erfolgt in Testgruppen, die aus jeweils drei Tieren bestehen. Das erste Tier erhält lediglich das Vehikel, um die spontane Adhäsion zu bestimmen. Das zweite Tier erhält lediglich die Leukozyten-Stimulation zur Bestimmung der pathogenen Kontrolle. Das dritte Tier erhält Leukozyten-Stimulanz und Testsubstanz.

Die Zahl der adhäsierenden Leukozyten in der pathogenen Kontrolle wird gleich 100 % gesetzt. Die prozentuale Änderung der Leukozyten-Adhäsion bei Testsubstanz Gabe gegenüber der pathogenen Kontrolle gibt die Wirksamkeit einer Testsubstanz an.

## Patentansprüche

1. Verfahren zur Herstellung eines physiologisch verträglichen und physiologisch abbaubaren Kohlenhydratrezeptorblockers auf Polymerbasis bestehend aus
a) einer hydrophilen, biodegradablen Polymereinheit,
b) wenigstens einer Di- oder Oligosaccharideinheit und
c) wenigstens einem bifunktionellen Spacer, über welchen die Di- oder Oligosaccharideinheiten mit der Polymereinheit verknüpft sind,
dadurch gekennzeichnet, daß durch chemische Verknüpfung eines Mono- oder Oligosaccharids, des Spacers und des hydrophilen, biodegradablen Polymers zunächst ein Akzeptor hergestellt wird, wonach ein oder mehrere weitere Monosaccharidbausteine durch enzymatische Glycosylierung angefügt werden.

2. Verfahren nach Anspruch 1, dadurch gekennzeichnet, daß die enzymatische Glycosylierung des Akzeptors in homogener, wäßriger Phase erfolgt.

3. Verfahren nach Anspruch 2, dadurch gekennzeichnet, daß die enzymatische Glycosylierung des Akzeptors mittels nucleotidaktivierten Kohlenhydraten als Donoren und Glycosyltransferasen erfolgt.

4. Verfahren nach Anspruch 3, dadurch gekennzeichnet, daß die enzymatische Glycosylierung des Akzeptors in einem der jeweiligen Glycosyltransferase angepaßten Puffersystem erfolgt.

5. Verfahren nach Anspruch 4, dadurch gekennzeichnet, daß das Puffersystem in einer Konzentration von 0,01 M bis 1 M vorliegt.

6. Verfahren nach Anspruch 4 oder 5, dadurch gekennzeichnet, daß das Puffersystem die zur Aktivierung der jeweiligen Glycosyltransferase notwendigen Kationen enthält.

7. Verfahren nach einem der Ansprüche 2 bis 6, dadurch gekennzeichnet, daß der pH-Wert des wäßrigen Mediums zwischen 6,0 und 8,5 liegt.

8. Verfahren nach einem der Ansprüche 3 bis 7, dadurch gekennzeichnet, daß bei äquimolarem oder überschüssigem Zusatz des Donors dem Reaktionsmedium alkalische Phosphatase zugesetzt wird.

9. Verfahren nach einem der Ansprüche 3 bis 8, dadurch gekennzeichnet, daß zu dem im wäßrigen Puffersystem gelösten Akzeptor und dem Nucleotid-aktivierten Kohlenhydrat 0,01 bis 10 Units der Glycosyltransferase gegeben werden.

10. Verfahren nach einem der Ansprüche 2 bis 9, dadurch gekennzeichnet, daß die enzymatische Glycosylierung 1 bis 5 Tage bei 10 bis 40°C durchgeführt wird.

11. Verfahren nach einem der Ansprüche 1 bis 10, dadurch gekennzeichnet, daß als hydrophiles, biodegradables Polymer ein Polycarbonat, Polyester, Polyamid, Polyanhydrid, Polyiminocarbonat, Polyanhydrid, Polyorthoester, Polydioxanon, Polyphosphazen, Polyhydroxycarbonsäure, Polyaminosäure oder ein Polysaccharid verwendet wird.

12. Verfahren nach Anspruch 11, dadurch gekennzeichnet, daß als hydrophiles, biodegradables Polymer eine Polyaminosäure mit einem Molekulargewicht kleiner oder gleich 70 kD, welche als Polyamid oder Polyanhydrid vorliegt, eingesetzt wird.

13. Verfahren nach Anspruch 12, dadurch gekennzeichnet, daß die Polyaminosäure Poly-α,β-(2-hydroxyethyl)-D,L-aspartamid, Poly-D,L-succinimid, Polyglutamat, Poly-L-Lysinmethylesterfumaramid oder ein Copolymer dieser Polyaminosäuren ist.

14. Verfahren nach einem der Ansprüche 1 bis 13, dadurch gekennzeichnet, daß der Spacer des Akzeptors Formel I hat
(Mono- oder Oligosaccharid)-O-[Q¹-(CH₂)ₚ-Q²]ᵣ-(Polymereinheit) I,
worin bedeuten
Q¹ -CH₂- oder 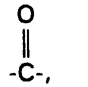
Q² -NH- oder 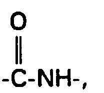
p eine ganze Zahl von 1 bis 6 und
r 1 oder 2.

## Claims

1. A process for preparing a physiologically tolerated and physiologically degradable polymer-based carbohydrate receptor blocker consisting of
a) a hydrophilic, biodegradable polymer unit,
b) at least one di- or oligosaccharide unit and
c) at least one bifunctional spacer by which the dior oligosaccharide units are linked to the polymer unit,
which comprises initially preparing an acceptor by chemical linkage of a mono- or oligosaccharide, of the spacer and of the hydrophilic, biodegradable polymer, after which one or more other monosaccharide units are attached by enzymatic glycosylation.

2. The process as claimed in claim 1, wherein the enzymatic glycosylation of the acceptor takes place in homogeneous aqueous phase.

3. The process as claimed in claim 2, wherein the enzymatic glycosylation of the acceptor takes place with the aid of nucleotide-activated carbohydrates as donors and glycosyltransferases.

4. The process as claimed in claim 3, wherein the enzymatic glycosylation of the acceptor takes place in a buffer system appropriate for the particular glycosyltransferase.

5. The process as claimed in claim 4, wherein the buffer system has a concentration of 0.01 M to 1 M.

6. The process as claimed in claim 4 or 5, wherein the buffer system contains the cations necessary for activating the particular glycosyltransferase.

7. The process as claimed in any of claims 2 to 6, wherein the pH of the aqueous medium is between 6.0 and 8.5.

8. The process as claimed in any of claims 3 to 7, wherein alkaline phosphatase is added to the reaction medium when the donor is added in equimolar amount or excess.

9. The process as claimed in any of claims 3 to 8, wherein 0.01 to 10 units of the glycosyltransferase are added to the acceptor dissolved in the aqueous buffer system and to the nucleotide-activated carbohydrate.

10. The process as claimed in any of claims 2 to 9, wherein the enzymatic glycosylation is carried out at 10 to 40°C for 1 to 5 days.

11. The process as claimed in any of claims 1 to 10, wherein a polycarbonate, polyester, polyamide, polyanhydride, polyiminocarbonate, polyorthoester, polydioxanone, polyphosphazene, polyhydroxy-carboxylic acid, polyamino-acid or a polysaccharide is used as hydrophilic, biodegradable polymer.

12. The process as claimed in claim 11, wherein a polyamino-acid with a molecular weight less than or equal to 70 kD, which is in polyamide or polyanhydride form, is used as hydrophilic, biodegradable polymer.

13. The process as claimed in claim 12, wherein the polyamino-acid is poly-α,β-(2-hydroxyethyl)-D,L-aspartamide, poly-D,L-succinimide, polyglutamate, poly-L-lysine methyl ester fumaramide or a copolymer of these polyamino-acids.

14. The process as claimed in any of claims 1 to 13, wherein the spacer of the acceptor has formula I
(mono- or oligosaccharide)-O-[Q¹-(CH₂)ₚ-Q²]ᵣ-(polymer unit) I,
in which
Q¹ is -CH₂- or 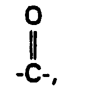
Q² is -NH- or 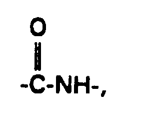
p is an integer from 1 to 6 and
r is 1 or 2.

## Revendications

1. Procédé pour la préparation d'un bloquant de récepteur de carbohydrate à base de polymère dégradable physiologiquement et toléré physiologiquement, constitué de
a) une unité polymère hydrophile biodégradable,
b) d'au moins une unité de di- ou d'oligosaccharide, et
c) d'au moins un spacer bifonctionnel par l'intermédiaire duquel sont couplées les unités de di- ou d'oligosaccharides à l'unité polymère,
caractérisé en ce que l'on prépare par couplage chimique d'un mono- ou oligosaccharide, du spacer et du polymère hydrophile biodégradable, d'abord un accepteur, ensuite on ajoute un ou plusieurs autres éléments de construction monosaccharides par glycosylation enzymatique.

2. Procédé selon la revendication 1, caractérisé en ce que l'on effectue la glycosylation enzymatique de l'accepteur en phase aqueuse homogène.

3. Procédé selon la revendication 2, caractérisé en ce que l'on effectue la glycosylation enzymatique de l'accepteur à l'aide de carbohydrate activé par des nucléotides en tant que donneurs et de glycosyltransférases.

4. Procédé selon la revendication 3, caractérisé en ce que l'on effectue la glycosylation enzymatique de l'accepteur dans un système de tampon adapté à la glycosyltransférase respective.

5. Procédé selon la revendication 4, caractérisé en ce que le système de tampon se présente dans une concentration de 0,01 M à 1 M.

6. Procédé selon la revendication 4 ou 5, caractérisé en ce que le système de tampon contient le cation nécessaire pour l'activation de la glycosyltransférase respective.

7. Procédé selon l'une des revendications 2 à 6, caractérisé en ce que la valeur du pH du milieu aqueux est comprise entre 6,0 et 8,5.

8. Procédé selon l'une des revendications 3 à 7, caractérisé en ce que l'on ajoute au milieu réactionnel la phosphatase alcaline pour un ajout équimolaire ou en excès du donneur.

9. Procédé selon l'une des revendications 3 à 8, caractérisé en ce que l'on ajoute à l'accepteur dissous dans le système de tampon aqueux et au carbohydrate activé par des nucléotides de 0,01 à 10 unités de glycosyltransférase.

10. Procédé selon l'une des revendications 2 à 9, caractérisé en ce que l'on met en oeuvre la glycosylation enzymatique pendant 1 à 5 jours, à une température de 10 à 40 °C.

11. Procédé selon l'une des revendications 1 à 10, caractérisé en ce que l'on utilise en tant que polymère biodégradable hydrophile un polycarbonate, polyester, polyamide, polyanhydride, polyiminocarbonate, polyanhydride, polyortoester, polydioxanone, polyphosphazène, acide polyhydroxycarboxylique, polyamino-acide, ou un polysaccharide.

12. Procédé selon la revendication 11, caractérisé en ce que l'on utilise en tant que polymère biodégradable hydrophile un polyamino-acide avec une masse molaire inférieure ou égale à 70 kD, lequel se présente sous forme de polyamide, ou de polyanhydride.

13. Procédé selon la revendication 12, caractérisé en ce que le polyamino-acide est le Poly-α,β-(2-hydroxyéthyl)-D,L-aspartamide, Poly-D,L-succinimide, Polyglutamate, Poly-L-lysineméthylesterfumaramide, ou un copolymère de ce polyamino-acide.

14. Procédé selon l'une des revendications 1 à 13, caractérisé en ce que le spacer de l'accepteur répond à la formule I
(Mono- ou oligosaccharide)-O-[Q¹-(CH₂)ₚ-Q²]ᵣ-(unité polymère) I,
dans laquelle
Q¹ représente -CH₂- ou 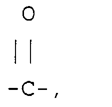
Q² représente -NH- ou 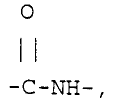
p est un nombre entier de 1 à 6, et
r vaut 1 ou 2.
